# EUROPEAN PATENT APPLICATION

(11) **EP 4 826 928 A1**
(43) Date of publication of application: **21.10.2026**
(21) Application number: 24903733.4
(22) Date of filing: 11.12.2024
(51) Int. Cl.: H01S 5/02208, A61B 18/24, H01S 5/0225, H01S 5/02255, H01S 5/0233, H01S 5/024

(54) **LIGHT IRRADIATION DEVICE AND IN-VIVO LIGHT IRRADIATION ASSEMBLY USING SAME**

(30) Priority: 15.12.2023 JP 2023211661
(71) Applicant: NICHIA CORPORATION, Anan-shi, Tokushima 774-8601 (JP)
(72) Inventor: MIYATA, Tadaaki, Anan-shi, Tokushima 774-8601 (JP)
(74) Representative: Betten & Resch
(86) International application number: PCT/JP2024/043896
(87) International publication number: WO 2025/127080

(57) **Abstract**

A small light irradiation device having heat dissipation and electrical insulation properties will be provided. The light irradiation device includes: a light-emitting element configured to emit light having a predetermined wavelength; a support body on which the light-emitting element is mounted; an insulated wire electrically connected to the light-emitting element; and an insulating layer integrally covering an outer surface of a structure including the light-emitting element, the support body, and the insulated wire.

## Description

### Technical Field

The present disclosure relates to a light irradiation device and an in-vivo light irradiation assembly using the same.

### Background Art

Endoscopes are used for diagnoses and treatments in a biological body. A treatment using a combination of an endoscope and laser irradiation is also performed. In photoimmunotherapy that has been attracting attention in recent years, when an affected area is on the body surface or in a region shallow from the body surface, the body surface is irradiated with light having a specific wavelength that reacts with a photosensitizer accumulated in cancer cells. When the affected area is located at a position deep from the body surface, the affected area in the body is irradiated with laser light by puncturing. Specifically, an optical fiber is inserted into a blood vessel, a digestive tract, or other lumen, and the affected area in the biological body is irradiated with laser light having a specific wavelength. A deployable balloon illumination for endoscopy has been proposed (for example, see Patent Document 1).

### Citation List

### Patent Literature

Patent Document 1: JP 2022-535144 T

### SUMMARY OF INVENTION

### Technical Problem

The configuration using the optical fiber is weak against bending, and optical loss increases in proportion to the length of the optical fiber. An ultra-small laser light source that can be inserted into the vicinity of an affected area in a body without an optical fiber is desired. According to one aspect of the present disclosure, a small light irradiation device having heat dissipation and electrical insulation properties can be provided.

### Solution to Problem

In one embodiment, a light irradiation device includes: a light-emitting element configured to emit light having a predetermined wavelength; a support body on which the light-emitting element is mounted; an insulated wire electrically connected to the light-emitting element; and an insulating layer integrally covering an outer surface of a structure including the light-emitting element, the support body, and the insulated wire. Advantageous Effects of Invention

A small light irradiation device having heat dissipation and electrical insulation properties can be realized.

### BRIEF DESCRIPTION OF DRAWINGS

FIG. 1 is a diagram illustrating an example of an in-vivo light irradiation assembly using a light irradiation device of a first embodiment.
FIG. 2A is a schematic view illustrating a configuration example of the light irradiation device of the first embodiment.
FIG. 2B is a schematic view illustrating another configuration example of the light irradiation device of the first embodiment.
FIG. 3 is a perspective view of a main body of a light irradiation device body before being sealed with an insulating layer.
FIG. 4 is a schematic view of a light-emitting element mounted on a support body.
FIG. 5 is a schematic view illustrating an example in which the support body and a light-emitting element are layered.
FIG. 6 is a diagram illustrating an example of an in-vivo light irradiation assembly using a light irradiation device of a second embodiment.
FIG. 7 is a diagram illustrating an example of arrangement of an optical element of the light irradiation device of the second embodiment.
FIG. 8 is a schematic view of a light irradiation device of a third embodiment.
FIG. 9 is a schematic view illustrating an example of a light-emitting element used in the third embodiment.
FIG. 10A is a perspective view of a support body used in the third embodiment on a light-emitting element mounting surface side.
FIG. 10B is a perspective view of the support body used in the third embodiment on a back surface side.
FIG. 11 is a diagram illustrating electrical characteristics and optical output characteristics of a light irradiation device of an embodiment.
FIG. 12 is a diagram illustrating a thermal resistance of the light irradiation device of the embodiment.

### DESCRIPTION OF EMBODIMENTS

Hereinafter, an embodiment for carrying out the present disclosure is described with reference to the drawings. The following description is intended to embody technical concepts of the present disclosure, and the present disclosure is not limited to the following unless specifically stated. In each drawing, members having identical functions may be denoted by the same reference characters. In consideration of the ease of explanation or understanding of the gist, the embodiments may be illustrated separately for convenience, but the partial substitutions or combinations of the configurations illustrated in different embodiments and examples are possible. In the embodiments to be described below, configurations different from the embodiment described before will be mainly described, and redundant descriptions of that are common with the embodiment described before will sometimes be omitted. The size, positional relationship, and other features of members illustrated in the drawings may be exaggerated in order to clarify explanation.

### First Embodiment

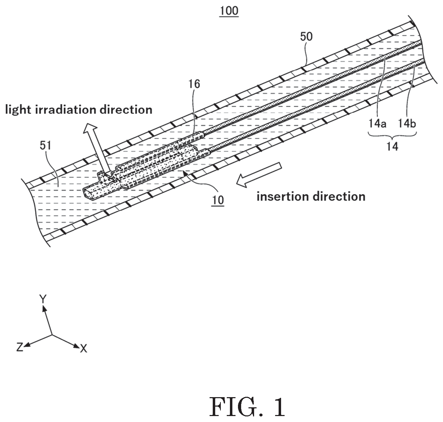

FIG. 1 is a diagram illustrating an example of an in-vivo light irradiation assembly 100 using a light irradiation device 10 of a first embodiment. The in-vivo light irradiation assembly 100 includes a catheter 50 and the light irradiation device 10 inserted into the catheter 50, and the inside of the catheter 50 is filled with a coolant 51 at least during use. The in-vivo light irradiation assembly 100 is used for treatment, diagnosis, sensing, and the like. The light irradiation device 10 is inserted into the catheter 50 in an "insertion direction" indicated by a white arrow in the drawing, for example, and is introduced into a biological body. Because the light irradiation device 10 is a heat generating element, the light irradiation device 10 is used in a state in which the coolant 51 is supplied into the catheter 50 and a heat generating portion is cooled. Therefore, a tip end side of the light irradiation device 10 is integrally covered with an insulating layer 16. The coolant 51 is, for example, physiological saline, blood, lymph, or the like, and the coolant temperature is, for example, about 10 °C to 36 °C. Because the light irradiation device 10 is inserted into the vicinity of a target site such as an affected area in the biological body by using the catheter 50, the affected area such as a tumor can be directly irradiated with light without using an optical fiber, or the target site for diagnosis, sensing, or the like can be examined and confirmed.

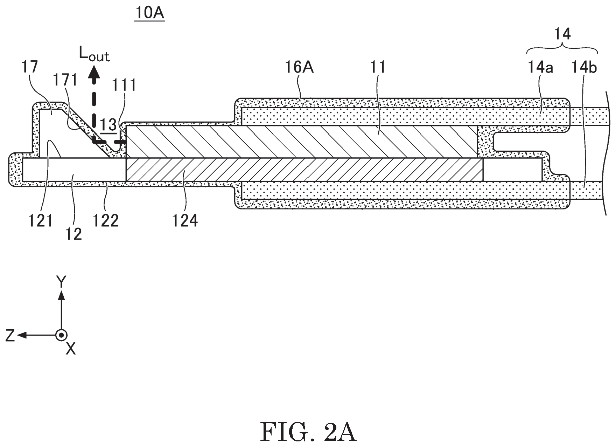

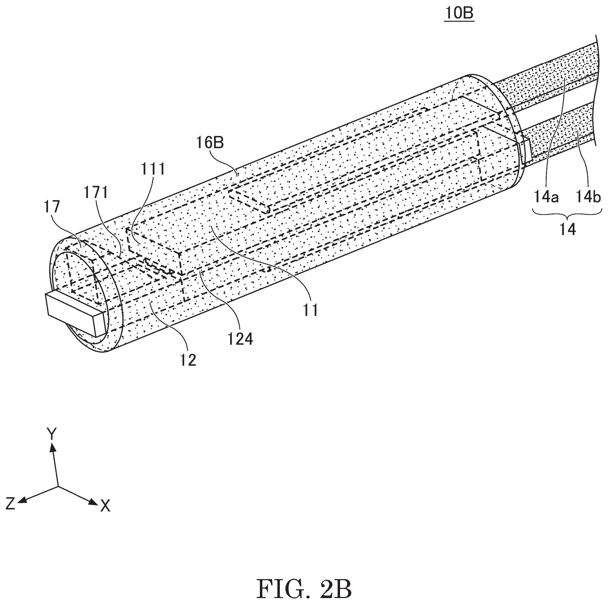

FIGS. 2A and 2B are schematic views illustrating a configuration example of the light irradiation device 10 of the first embodiment illustrated in FIG. 1. In both a light irradiation device 10A in FIG. 2A and a light irradiation device 10B in FIG. 2B, a light-emitting element, a support body on which the light-emitting element is mounted, and an insulated wire electrically connected to the light-emitting element are integrally covered with an insulating layer, but the covering state of the insulating layer is different between them. In the coordinate systems of FIGS. 2A and 2B, an optical axis direction is defined as a Z direction, a direction in which the light-emitting element is mounted on the support body is defined as a Y direction, and a direction orthogonal to the Z direction and the Y direction is defined as an X direction. The light irradiation device 10A illustrated in FIG. 2A includes a light-emitting element 11 that emits light having a predetermined wavelength, an optical component 17 on which the light emitted from the light-emitting element 11 is incident, a support body 12 on which the light-emitting element 11 and the optical component 17 are mounted, insulated wires 14 electrically connected to the light-emitting element 11, and an insulating layer 16A that integrally covers an outer surface of a structure that includes the light-emitting element 11, the optical component 17, the support body 12, and the insulated wires 14. A space 13 is provided between a light-emitting surface 111 of the light-emitting element 11 and a light incident surface 171 of the optical component 17. By providing the space 13 and shortening the distance over which the light emitted from the light-emitting element 11 passes through the insulating layer, the influence of refraction of light by the insulating layer can be reduced, so that the spread of the light emitted from the light-emitting element 11 is easily controlled. The insulating layer 16A is a coating formed by applying a polysilazane solution and converting the applied coating into silica (hereinafter, referred to as "polysilazane coat"), and is light-transmissive to the light emitted from the light-emitting element 11. By employing the polysilazane coat, the insulation property can be ensured, while the thickness of the insulating layer is reduced to improve the heat dissipation property. The light-emitting surface 111 of the light-emitting element 11 and the light incident surface 171 of the optical component 17 face each other with the space 13 present therebetween. The light emitted from the light-emitting surface 111 passes through the insulating layer 16A, the space 13, and the insulating layer 16A, and enters the light incident surface 171 of the optical component 17. The insulating layer 16A in FIG. 2A seals the layered structure along the surface shapes of the support body 12 and the components mounted on the support body 12. The space 13 may be filled with the coolant 51 when the light irradiation device 10A is inserted into the catheter 50 and used. In this case, the light passes through the coolant 51 in the space 13 and enters the light incident surface 171 of the optical component 17.

In this configuration example, the longitudinal direction of the support body 12 is parallel to the Z direction. The light irradiation device 10 of the first embodiment is inserted into the catheter 50 in the + Z direction, and the insulated wires 14 including a first insulated wire 14a and a second insulated wire 14b extends in the - Z direction. The support body 12 is made of an insulating material such as silicon (Si), aluminum nitride (AlN), silicon nitride (SiN), sapphire (Al₂O₃), glass, quartz, or ceramic, and the light-emitting element 11 is mounted on a light-emitting element mounting surface 121 of the support body 12.

The optical component 17 is mounted together with the light-emitting element 11 on the light-emitting element mounting surface 121 of the support body 12. The light incident surface 171 of the optical component 17 is inclined with respect to the light-emitting element mounting surface 121. Thus, the light incident surface 171 of the optical component 17 functions as a reflective surface, and output light Lout of the light irradiation device 10A is extracted in the direction of the broken line arrow. In the configuration example of FIG. 2A, the optical component 17 is a reflective member that guides the light emitted from the light-emitting element 11 in a direction intersecting the light-emitting element mounting surface 121 of the support body 12 (for example, the + Y direction). Thus, the light can be emitted in a circumferential direction of the light irradiation device. The optical component 17 may be a mirror that reflects light incident on the light incident surface 171. The mirror may include a reflective layer of metal and/or dielectric multilayer film. Thus, light can be efficiently reflected.

The insulated wires 14 include the first insulated wire 14a and the second insulated wire 14b.
The first insulated wire 14a is electrically connected to one of electrodes of the light-emitting element 11 (also referred to as a first electrode) on the side of the light-emitting element mounting surface 121, which is a first surface of the support body 12 on which the light-emitting element 11 is mounted. The second insulated wire 14b is electrically connected to the other electrode of the light-emitting element 11 (also referred to as a second electrode) on the side of a back surface 122 (also referred to as a second surface) opposite to the light-emitting element mounting surface 121. Using the insulated wires allows for supplying electricity to the light-emitting element 11. One of the features of the light irradiation device 10A is that electrical connection portions between the first insulated wire 14a and the light-emitting element 11 and between the second insulated wire 14b and the light-emitting element 11 together with the support body 12, the light-emitting element 11, and the optical component 17 are integrally sealed with the insulating layer 16A. The thickness of the insulating layer 16A is smaller than the thickness of the light-emitting element 11. By integrally covering the light irradiation device 10A with the insulating layer 16 having a smaller thickness than the light-emitting element 11, a small light irradiation device 10A that ensures an electrical insulation property can be realized, and insertion into the catheter 50 is facilitated. In addition, by making the thickness of the insulating layer 16A smaller than the thickness of the light-emitting element, the thermal resistance of the insulating layer 16 can be reduced, and the heat dissipation property can be improved.

The width of the support body 12 in the X direction is a width that allows the support body 12 to be easily inserted into the catheter 50, but may be set as wide as possible within a range in which the support body 12 can be smoothly inserted into the catheter 50 from the viewpoint of increasing a cooling area (that is, heat dissipation area) with the coolant 51. The thickness of the support body 12 in the Y direction is a thickness by which the light-emitting element 11 can be stably supported, and the support body 12 with the light-emitting element 11 mounted thereon can be smoothly inserted into the catheter 50. As an example, the thickness of the support body 12 is in a range from about 0.075 to 0.3 mm.

The insulating layer 16A for integrally sealing the support body 12 and the components on the support body 12 is a polysilazane coat, or insulating resins such as epoxy resins, silicone resins, acrylic resins, or thermoplastic resins. By integrally sealing the support body 12 and the components with the insulating layer, that is, by sealing the support body 12 and the components in a single step, the steps can be simplified. When these resins are used, insulating resins that are biocompatible and have high thermal conductivity are preferably used for the insulating layer 16A. As such an insulating resin, polycarbonate (PC), polyethylene terephthalate (PET), polyimide (PI), polyurethane, polyester imide, or the like, whose components and composition are adjusted to mitigate adverse effects on a biological body, can be used. The insulating layer 16A may be obtained by providing a biocompatible resin layer on the surface of a polysilazane coat. The insulating layer 16A isolates the electrical connection portions between the light-emitting element 11 and the first insulated wire 14a and between the light-emitting element 11 and the second insulated wire 14b from the coolant 51. While maintaining this sealed state, the first insulated wire 14a and the second insulated wire 14b are extended outward from the insulating layer 16A in the - Z direction.

The first insulated wire 14a and the second insulated wire 14b are cooled by the coolant 51 and also function as a heat dissipation material. The insulated wires 14 are, for example, an enameled wire.

A metal wire of a good conductor such as Cu or Ni is insulation-coated with an insulating coating of polyurethane. Instead of polyurethane, coating of polyester, polyesterimide, polyamideimide, polyimide, or the like, which is an insulating resin, may be applied. By using the insulating coating, which is the surface coating on the insulated wire 14, as a heat dissipation material, heat generated from the light-emitting element 11 can be released to the outside. When rectangular enameled wires are used as the first insulated wire 14a and the second insulated wire 14b, the wires are wide wires, and the bonding area with the support body 12 can be increased. In addition, when the area of a circumscribed circle is equal, the square enameled wire can be made smaller in the area of the circumscribed circle than a round enameled wire, and thus can be made smaller in size.

The light-emitting element 11 is, for example, an edge-emitting laser element, and resonates in a direction parallel to a Z axis. When an edge-emitting laser element is used as the light-emitting element 11, one end surface thereof in the resonance direction serves as the light-emitting surface 111. The optical component 17 is disposed on the light-emitting element mounting surface 121 of the support body 12 to face the light-emitting surface 111 of the light-emitting element 11, and guides light emitted from the edge-emitting laser element in a direction intersecting the light-emitting element mounting surface 121. The direction intersecting the light-emitting element mounting surface 121 is any of all directions except for the direction parallel to the light-emitting element mounting surface 121, but may be, for example, in a range of 50° to 130° relative to the light-emitting element mounting surface 121. It may be a direction perpendicular to the light-emitting element mounting surface 121. The expression "perpendicular" direction does not need to be exactly a right angle to the light-emitting element mounting surface 121, and includes a range of 90° ± 5° in consideration of manufacturing errors and surface states of the support body 12 and the optical component 17. The emitted light from the light-emitting element 11 is reflected by the light incident surface 171 of the optical component 17 and is emitted in a predetermined direction. When a polysilazane coat is used for the insulating layer 16A, light may be reflected by the polysilazane coat. The optical component 17 may be made up of a combination of a first optical element that controls the divergence angle of light emitted from the light-emitting element 11 and a second optical element having a reflection function, as long as a predetermined space 13 is maintained between the optical component 17 and the light-emitting surface 111 of the light-emitting element 11.

In the configuration example of FIG. 2A, the first insulated wire 14a and the second insulated wire 14b are connected to an upper surface of the light-emitting element 11 and the back surface 122 of the support body 12, respectively, so as not to hinder light emission from the light-emitting element 11 in the Z direction or reflection by the optical component 17. A conductive layer 124 is formed on at least a part of the surface of the support body 12. In the example of FIG. 2A, the support body 12 has the light-emitting element mounting surface 121, the back surface 122, and the conductive layer 124 formed on at least a part of a lateral surface connecting the light-emitting element mounting surface 121 and the back surface 122. Thus, the first insulated wire 14a is electrically connected directly or indirectly to one of the electrodes of the light-emitting element 11 on the side of the light-emitting element mounting surface 121 of the support body 12. The second insulated wire 14b is electrically connected directly or indirectly to the other electrode of the light-emitting element 11 via the conductive layer 124, which is formed on the support body 12, on the back surface 122 of the support body 12.

The configurations of the light-emitting element 11 and the optical component 17 mounted on the support body 12 of the light irradiation device 10B in FIG. 2B and the insulated wires 14 electrically connected to the light-emitting element 11 are the same as those of the light irradiation device 10A, but the state of covering with an insulating layer 16B is different from that in FIG. 2A. The thickness of the insulating layer 16B may be at least partially greater than the thickness of the light-emitting element 11. In this case, the space between the light-emitting surface 111 of the light-emitting element 11 and the light incident surface 171 of the optical component 17 may be filled with the insulating layer 16B. The light emitted from the light-emitting surface 111 of the light-emitting element 11 passes through the insulating layer 16B and enters the light incident surface 171 of the optical component 17. The material of the insulating layer 16B may be the same as that of the insulating layer 16A described above. With the increase in the thickness of the insulating layer 16B, the support body 12, the light-emitting element 11, the optical component 17, and the insulated wires 14 can be stably held in their entirety, and optical coupling portions and electrical connection portions are securely inhibited from coming into contact with the coolant.

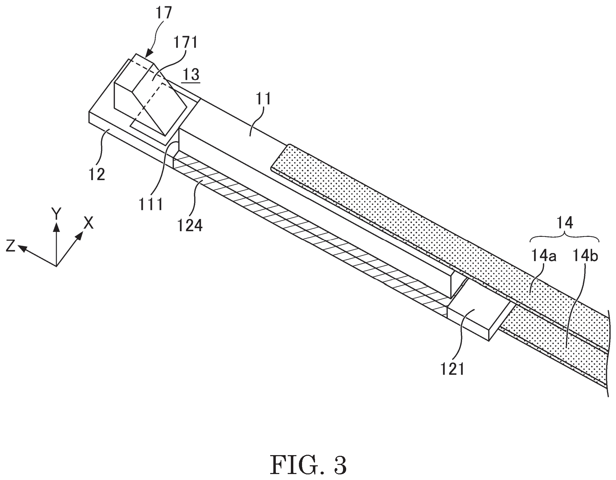

FIG. 3 is a perspective view of a main body of the light irradiation device before being sealed with the insulating layer 16 (or 16A or 16B). The predetermined space 13 is provided between the light-emitting surface 111 of the light-emitting element 11 and the light incident surface 171 of the optical component 17. The light emitted from the light-emitting element 11 passes through the space 13, enters the optical component 17, and is reflected in a predetermined direction. The conductive layer 124 covers at least a part of the light-emitting element mounting surface 121, at least a part of the lateral surface of the support body 12, and at least a part of the back surface 122 of the support body 12. The upper surface of the light-emitting element 11 is connected to the first insulated wire 14a, and a bottom surface of the light-emitting element 11 is connected to the conductive layer 124 formed on the light-emitting element mounting surface 121.

A part of the insulating coating on the first insulated wire 14a is removed, and the internal metal wire is connected to one of the electrodes of the light-emitting element 11. A part of the insulating coating on the second insulated wire 14b is removed, and the internal metal wire is electrically connected to the other electrode of the light-emitting element 11. In the example of FIG. 3, the conductive layer 124 is formed from the light-emitting element mounting surface 121 of the support body 12 to the back surface 122 through the lateral surface thereof, and the metal wire inside the second insulated wire 14b is connected to the conductive layer 124 on the back surface 122 of the support body 12. This can establish electrical connection between the other electrode of the light-emitting element 11 and the second insulated wire 14b. By providing the conductive layer 124 for electrical connection on the surface of the support body 12, the conductive layer 124 can be used as a heat dissipation member.

As described above, the optical component 17 is mounted on the support body 12 and faces the light-emitting surface 111 of the light-emitting element 11. The main body of the optical component 17 is made of a dielectric such as glass, and a thin film of silver is formed on the light incident surface 171; however, the optical component 17 is not limited to this example. A thin film of aluminum, aluminum alloy, gold, nickel, platinum, or the like may be formed on the light incident surface (that is, the reflective surface) of the plastic main body. A dielectric multilayer film may be formed instead of the metal reflective film. The support body 12, the light-emitting element 11, the optical component 17, and a part of the insulated wire 14 are covered with the light-transmissive insulating layer 16A or 16B by a dipping method, a spraying method, or the like.

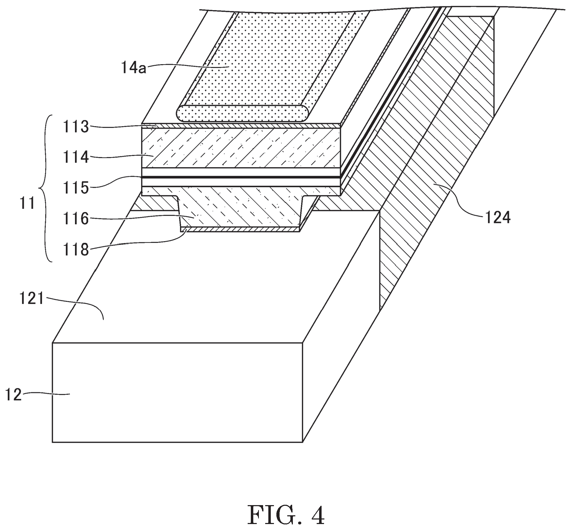

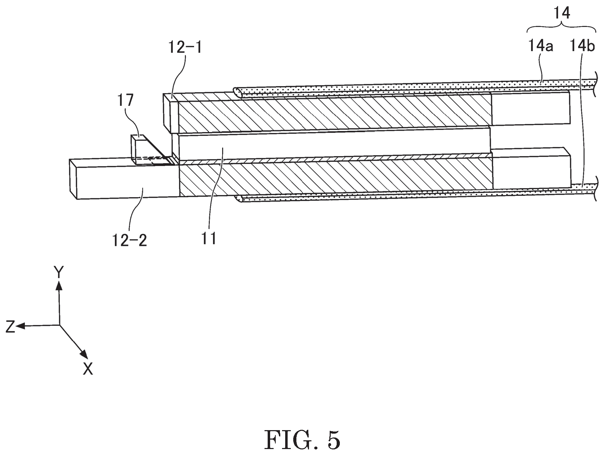

FIG. 4 is a schematic view of the light-emitting element 11 mounted on the support body 12. The light-emitting element 11 is, for example, an edge-emitting laser element having a ridge structure. The light-emitting element 11 includes an n-side electrode 113, an n-side semiconductor layer 114, an active layer 115, a p-side semiconductor layer 116, and a p-side electrode 118. The n-side semiconductor layer 114 may include an optical guide layer, a cladding layer, a contact layer, and the like doped with an n-type impurity. The p-side semiconductor layer 116 may include an optical guide layer, a cladding layer, a contact layer, and the like doped with a p-type impurity. The p-side electrode 118 is electrically connected to the first insulated wire 14a. When an ITO electrode is used as the p-side electrode 118, the p-side electrode 118 may also serve as a cladding layer. The p-side electrode 118 is electrically connected to the second insulated wire 14b via the conductive layer 124 formed on the support body 12. In the example of FIG. 4, the n-side electrode 113 and the first insulated wire 14a are directly connected, but may be indirectly connected. For example, a second support body may be disposed between the n-side electrode 113 and the first insulated wire 14a. As described below with reference to FIG. 5, the heat dissipation property can be improved by arranging the light-emitting element 11 between two support bodies 12.

The semiconductor materials of the light-emitting element 11 and their composition are designed to cause emission of laser light having a desired wavelength. When the light-emitting element 11 is used as an ultraviolet laser, a violet laser, a blue laser, and a green laser, a GaN-based material such as GaN, InGaN, and AlGaN is used. When the light-emitting element 11 is used as a red laser, an infrared laser, and a near-infrared laser, a GaAs-based material such as GaAs or AlGaAs or an InP-based material such as InAlGaP or GaInP is used. In consideration of sealing, together with the first insulated wire 14a and the second insulated wire 14b, the light-emitting element 11 mounted on the support body 12 with the insulating layer 16A or 16B, the ridge may be widened to secure the gain of the active layer 115. The width of the ridge may be designed to be, for example, 2 µm to 100 µm. The transverse mode may be a multimode or a single mode.

Unlike a fiber-type or external light source-type irradiation device, the light emitted by the light-emitting element 11 is linearly polarized light, and is reflected at the optical component 17 such as a mirror to travel in a different direction, so that the direction of the linearly polarized light after emission can be changed. This makes it possible to reduce the reflectance of light incident on a biological body and improve the transmittance of light to the biological body, for example. Even if laser light is in a specific polarization state upon being emitted, its polarization state may fluctuate or it may be depolarized, while propagating through a transmission medium such as an optical fiber. In the present embodiment, since the affected area in the body can be directly irradiated with light without using an optical fiber, a light can be irradiated to a target portion such as the affected area while maintaining a specific polarization state.

FIG. 5 is a schematic view illustrating an example in which the support body 12 and the light-emitting element 11 are layered. In this configuration example, the light-emitting element 11 is arranged between a first support body 12-1 and a second support body 12-2. The first support body 12-1 is in contact with the upper surface of the light-emitting element 11, and the second support body 12-2 is in contact with the bottom surface of the light-emitting element 11. By arranging the light-emitting element 11 between the first support body 12-1 and the second support body 12-2, heat can be efficiently released to the upper side and the lower side of the light-emitting element 11, and the heat dissipation property of the light irradiation device 10 can be improved. The rear end of at least one of the first support body 12-1 or the second support body 12-2 may extend in the - Z direction. In FIG. 5, it is assumed that light is extracted by the optical component 17 in a direction (for example, the Y direction) not parallel to the optical axis (Z axis); however, as will be described in a second embodiment, when light is extracted in a direction parallel to the light-emitting element mounting surface 121 of the support body 12, the tip end sides of the first support body 12-1 and the second support body 12-2 may be extended in the + Z direction. The entire structure of FIG. 5 is covered with the insulating layer 16A or 16B.

### Second Embodiment

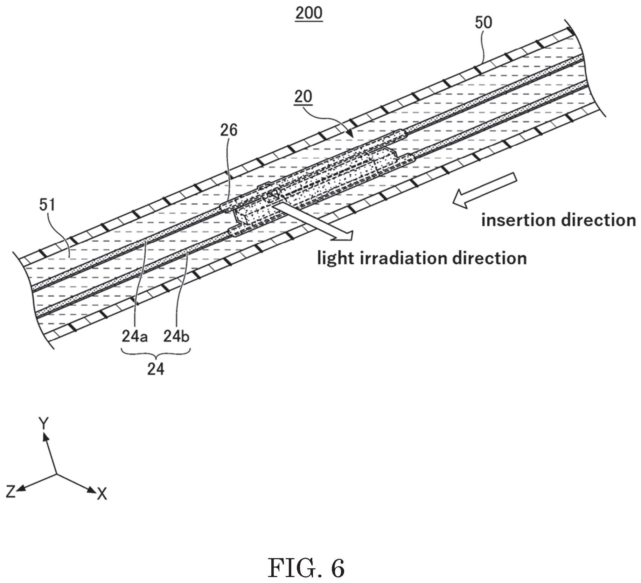

FIG. 6 is a diagram illustrating an example of an in-vivo light irradiation assembly 200 using a light irradiation device 20 of the second embodiment. The in-vivo light irradiation assembly 200 includes the catheter 50 and the light irradiation device 20 inserted into the catheter 50, and the inside of the catheter 50 is filled with the coolant 51 at least during use. The in-vivo light irradiation assembly 200 is used for treatment, diagnosis, sensing, and the like. Similarly to the light irradiation device 10 of the first embodiment, the light irradiation device 20 is inserted into the catheter 50 in the "insertion direction" indicated by a white arrow in the drawing, and is introduced into a biological body. The inside of the catheter 50 is filled with the coolant 51.

The light irradiation device 20 is used in a state in which the heat generating portion thereof is cooled. In the second embodiment, insulated wires 24 are extended outward from both sides of an insulating layer 26 along an optical axis to enhance the heat dissipation property. The insulated wires 24 include a first insulated wire 24a and a second insulated wire 14b electrically connected to the light-emitting element 11. The length of the insulated wire 24 extended outward from the insulating layer 26 in the + Z direction may be smaller than the length of the insulated wire 24 extended outward from the insulating layer 26 in the - Z direction. In order to realize this configuration, light emitted from the light-emitting element 11 is guided in a direction parallel to a light-emitting element mounting surface or in a direction in which the light is not obstructed by the first insulated wire 24a and the second insulated wire 24b. As illustrated in FIG. 2A, the insulating layer 26 of a small thickness may cover the support body 12 and the components mounted on the support body 12 along their surface shapes to improve the heat dissipation property. Alternatively, as illustrated in FIG. 2B, the insulating layer 26 may be partially thick to fill the regions between the components mounted on the support body 12 to ensure electrical insulation from the coolant 51 while reducing the thickness of the entire light irradiation device as much as possible.

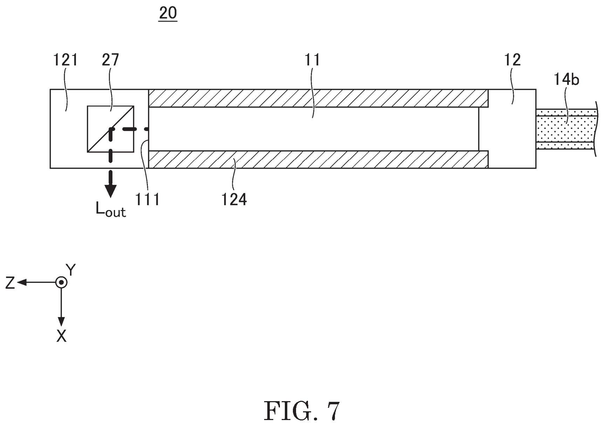

FIG. 7 is a diagram illustrating an arrangement example of an optical component 27 of the light irradiation device 20. In order to clearly illustrate the configuration, FIG. 7 illustrates a state before the first insulated wire 24a and the second insulated wire 24b are electrically connected to the light-emitting element 11. Light emitted from the light-emitting surface 111 of the light-emitting element 11 is reflected by the optical component 27 in a direction substantially parallel to the light-emitting element mounting surface 121, and output light Lout from the light irradiation device 20 is extracted in a direction of the broken line arrow. As long as the reflected light from the optical component 27 is not obstructed by the first insulated wire 24a or the second insulated wire 24b, the light may be reflected in a direction not parallel to the light-emitting element mounting surface 121.

In the actual light irradiation device 20, the light-emitting element 11, the optical component 27, and electrical connection portions between the light-emitting element 11 and the first insulated wire 24a and between the light-emitting element 11 and the second insulated wire 24b are sealed with the insulating layer 26 integrally with the support body 12. At least a portion of the insulating layer 26 between the light-emitting surface 111 of the light-emitting element 11 and a light incident surface 171 of the optical component is configured to transmit the light emitted from the light-emitting element 11.

The light-emitting element 11 is, for example, an edge-emitting laser element. The light-emitting element 11 may be a laser element having a wide ridge structure that is the same as or similar to that of the first embodiment. The direction parallel to the Z-axis of the light-emitting element 11 is the resonance direction. The optical component 27 is disposed on the light-emitting element mounting surface 121 of the support body 12, and guides light emitted from the light-emitting surface of the laser element in a direction parallel to the light-emitting element mounting surface 121 of the support body 12. The first insulated wire 24a and the second insulated wire 24b (see FIG. 6) extend in the + Z direction and the - Z direction with the light-emitting element 11 and the support body 12 arranged between the first insulated wire 24a and the second insulated wire 24b, so that the light from the light-emitting element 11 is reflected by the optical component 27 in such a direction that the light is not obstructed by the first insulated wire 24a or the second insulated wire 24b.

The optical component 27 reflects the light emitted from the light-emitting surface of the light-emitting element 11 in, for example, the X direction parallel to the light-emitting element mounting surface 121. The expression "parallel" direction does not indicate that it need to be exactly parallel to the light-emitting element mounting surface 121, and may encompass deviation of about ± 10° including a surface state of the support body 12, a manufacturing error of the optical component 27, and the like. The laser light reflected by the optical component 27 may be extracted at any angle other than parallel as long as the laser light is not interfered with by the first insulated wire 24a or the second insulated wire 24b. The optical component 27 is preferably as thick as the light-emitting element 11 or thinner than the light-emitting element 11. The optical component is a surface-flattened mirror or prism, or a meta-polarization element having a metasurface element inserted therein. The optical component 27 may be an optical element having a lens function of controlling the divergence angle of the light emitted from the light-emitting element 11. The optical component 27 may be made up of two or more optical elements. For example, the optical component 27 may include an optical element having a lens function of controlling the divergence angle of the light emitted from the light-emitting element 11, and an optical element that reflects, at a predetermined angle, the light whose divergence angle has been controlled by the optical element.

The insulating layer 26 integrally seals a portion that needs to be electrically insulated in a state in which the first insulated wire 24a and the second insulated wire 24b are extended in the + Z direction and the - Z direction from both ends of the support body 12 in the optical axis direction. The electrical connection portions between the light-emitting element 11 and the first insulated wire 24a and between the light-emitting element 11 and the second insulated wire 24b, and at least the light-emitting surface 111 of the light-emitting element 11 and the light incident surface 171 of the optical component 17 are sealed by the insulating layer 26 and are isolated from the coolant 51. The insulating layer 26 is preferably a biocompatible resin having high thermal conductivity.

At least one of both ends of the support body 12 in the longitudinal direction (Z direction) may protrude from the insulating layer 26. The support body 12 protruding from the insulating layer 26 is in contact with the coolant 51, so that the heat dissipation property of the light irradiation device 20 is ensured. In addition, with the first insulated wire 24a and the second insulated wire 24b that are extended outward of both sides of the support body 12 in the longitudinal direction, the heat dissipation property is further improved when compared with the first embodiment. Similarly to the first embodiment, the overall thickness and width of the light irradiation device 20 are about 0.5 mm, and an ultra-small light irradiation device 20 that can be incorporated into the catheter 50 is realized. In the configuration of the second embodiment, the light-emitting element 11 may be arranged between two support bodies 12-1 and 12-2 as illustrated in FIG. 5. In the second embodiment, the light emitted from the light-emitting element 11 is extracted in a direction that allows for preventing light from being obstructed by the support body 12, and thus the heat dissipation property of the light irradiation device 20 can be further improved by arranging the light-emitting element 11 between the two support bodies 12-1 and 12-2.

### Third Embodiment

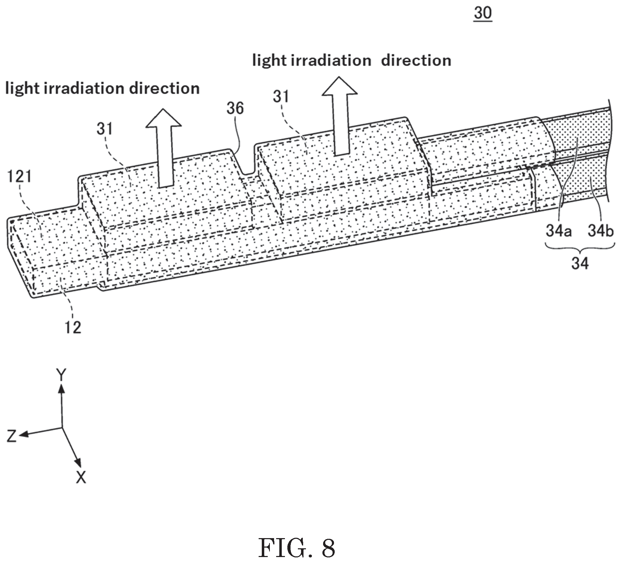

FIG. 8 is a schematic view of a light irradiation device 30 of the third embodiment. Similarly to the first embodiment and the second embodiment, the light irradiation device 30 is also used by being incorporated into the catheter 50 (see FIGS. 1 and 6), and the light irradiation device 30 and the catheter 50 can constitute an in-vivo light irradiation assembly. In the third embodiment, a vertical cavity surface emitting laser (VCSEL) is used as a light-emitting element 31.

The light irradiation device 30 includes two light-emitting elements 31 that emit light having a predetermined wavelength, the support body 12 on which the light-emitting elements 31 are mounted, and an insulated wire 34 electrically connected to the light-emitting elements 31. The insulated wire 34 includes a first insulated wire 34a connected to one of electrodes of each light-emitting element 31 and a second insulated wire 34b connected to the other electrode of the light-emitting element 31. A light-emitting surface of the light-emitting element 31, and an electrical connection portion between the light-emitting element 31 and the insulated wire 34 are sealed integrally with the support body 12 by an insulating layer 36. At least one of both ends of the support body 12 along the longitudinal direction (Z direction) may protrude from the insulating layer 36. At least a portion of the insulating layer 36 covering the light-emitting surface is light-transmissive. The thickness of the insulating layer 36 may be greater or smaller than the thickness of the light-emitting element 31.

The light-emitting surface of the VCSEL used as the light-emitting element 31 is parallel to the light-emitting element mounting surface 121 of the support body 12. The laser light emitted from the VCSEL is emitted in a direction perpendicular to the light-emitting element mounting surface 121 as indicated by a white arrow in the drawing. In this configuration, optical components such as mirrors are not required.

The first insulated wire 34a and the second insulated wire 34b are, for example, two-core enameled wires. The first insulated wire 34a is connected to one of the electrodes of each of the light-emitting elements 31 on the side of the light-emitting element mounting surface 121 of the support body 12.
The second insulated wire 34b is connected to the other electrode of each of the light-emitting elements 31 on the back surface (the surface on the side opposite to the light-emitting element mounting surface 121) of the support body 12. The first insulated wire 34a and the second insulated wire 34b are extended outward of the insulating layer 36 from at least one of both ends of the support body 12 in the longitudinal direction with the support body 12 interposed between the first insulated wire 34a and the second insulated wire 34b.

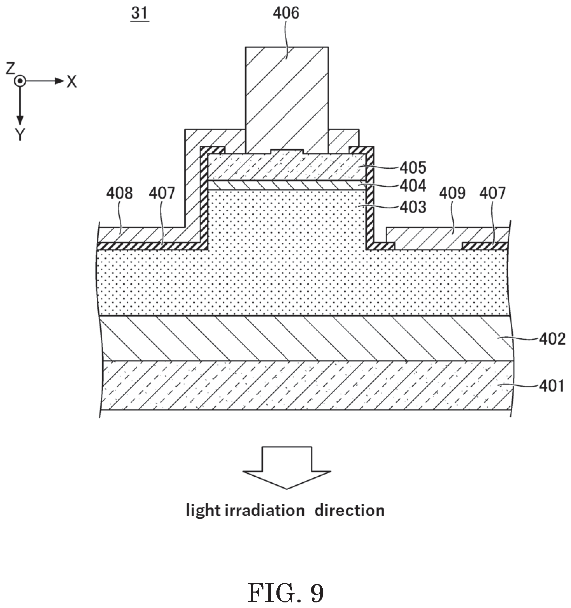

FIG. 9 is a schematic view illustrating an example of the light-emitting element 31 used in the third embodiment. The VCSEL as the light-emitting element 31 has a layered structure in which a semiconductor substrate 401, an n-side reflective film 402, an n-type semiconductor layer 403, an active layer 404, a p-type semiconductor layer 405, and a p-side reflective film 406 are layered in this order in the - Y direction. The light emission direction is the + Y direction. The conductivity types of the p-type and the n-type may be reversed. The semiconductor substrate 401 may be removed. The n-type semiconductor layer 403 includes a flat plate portion and a protruding portion protruding from the flat plate portion in the - Y direction. The active layer 404 is provided on an upper surface of the protruding portion of the n-type semiconductor layer 403. The p-type semiconductor layer 405 is provided on an upper surface of the active layer 404, and the p-side reflective film 406 is provided in a region of an upper portion of the p-type semiconductor layer 405, other than the peripheral region of the upper portion. A p-side contact layer may be provided between the p-type semiconductor layer 405 and the p-side reflective film 406.

The light-emitting element 31 includes an insulating layer 407 that covers an upper surface of the flat plate portion and a lateral surface of the protruding portion of the n-type semiconductor layer 403, a lateral surface of the active layer 404, and a lateral surface and a peripheral region of an upper surface of the p-type semiconductor layer 405. The light-emitting element 31 includes a p-side electrode 408 electrically connected to the p-type semiconductor layer 405, and an n-side electrode 409 electrically connected to the n-type semiconductor layer 403. The side on which the p-side electrode 408 and the n-side electrode 409 are provided is disposed on the light-emitting element mounting surface 121 of the support body 12. A conductive connection layer (or bump) to be connected to the light-emitting element mounting surface 121 of the support body 12 may be provided so that the height of the p-side electrode 408 and the n-side electrode 409 is aligned in the - Y direction. When the light-emitting element 31 is flip-chip mounted on the support body 12 with the conductive connection layer connected to the p-side electrode 408 and the n-side electrode 409, the p-side reflective film 406 does not interfere with the light-emitting element mounting surface of the support body 12.

The n-side reflective film 402 and the p-side reflective film 406 may each be formed of, for example, a distributed Bragg reflector (DBR). The DBR has a structure in which a plurality of high refractive index layers and a plurality of low refractive index layers are alternately layered. The DBR includes a wavelength region of high reflectance called a stop band. A center wavelength and a wavelength width of the stop band are determined by a refractive index and a thickness of the high refractive index layer and a refractive index and a thickness of the low refractive index layer. The reflectance in the stop band of the DBR increases with the refractive index difference between the high refractive index layer and the low refractive index layer and with the number of layers.

In the example illustrated in FIG. 9, a standing wave is formed between the n-side reflective film 402 and the p-side reflective film 406. A wavelength of the standing wave in the atmosphere is within the stop bands of the n-side reflective film 402 and the p-side reflective film 406, and this wavelength is an oscillation wavelength of the laser light. An integral multiple of a half of the oscillation wavelength is equal to an optical distance between reflective surfaces of the n-side reflective film 402 and the p-side reflective film 406 opposed to each other. The optical distance is a distance obtained by multiplying a distance in which light actually propagates through a certain medium by a refractive index of the medium. By applying a forward voltage between the p-side electrode 408 and the n-side electrode 409, current can be injected into the active layer 404. By the current injection, population inversion occurs in the active layer 404, and amplification of light by stimulated emission at the oscillation wavelength, that is, laser oscillation occurs. As described above, it is assumed that the p-side electrode 408 and n-side electrode 409 side of the VCSEL of the present embodiment is a mounting surface, and laser light is extracted from the semiconductor substrate 401 side.

The configuration of the VCSEL illustrated in FIG. 9 is an example. The components included in the VCSEL may be formed using known materials. The shape of some of the components included in the VCSEL may be changed, or the VCSEL may further include other components. Laser light may be extracted from the side opposite to the semiconductor substrate 401.

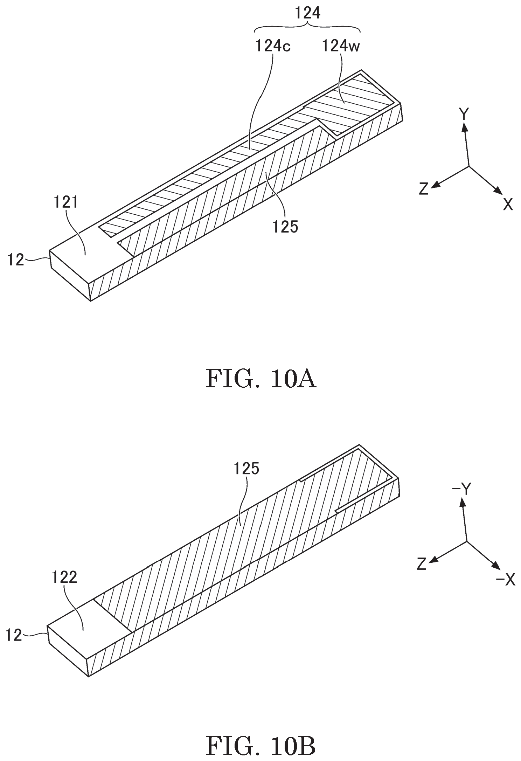

FIG. 10A is a perspective view of the support body 12 used in the third embodiment on the light-emitting element mounting surface 121 side. FIG. 10B is a perspective view of the support body on the back surface 122 side. The surface opposite to the light-emitting element mounting surface 121 is referred to as a "back surface". Conductive layers 124 and 125 are formed on the light-emitting element mounting surface 121 of the support body 12. The conductive layer 124 is formed on the light-emitting element mounting surface 121 of the support body 12, and is electrically insulated from the conductive layer 125. The conductive layer 124 has a connection region 124c connected to one of the electrodes of the light-emitting element 31, and a wide portion 124w that is wider than the connection region 124c at the end portion of the support body 12 on the - Z side. The wide portion 124w is used for electrical connection with the first insulated wire 34a. The conductive layer 125 is formed from the lateral surface of the support body 12 to the back surface 122.

The p-side electrode 408 and the n-side electrode 409 of the light-emitting element 31 are connected to the conductive layers 124 and 125 on the light-emitting element mounting surface 121, respectively, via the conductive connection layer (or bump). The wide portion 124w of the conductive layer 124 is electrically connected to the first insulated wire 34a. A part of the insulating coating on the first insulated wire 34a is removed, and the internal metal wire is connected to the conductive layer 124. On the back surface 122 of the support body 12, the conductive layer 125 is electrically connected to the second insulated wire 34b. A part of the insulating coating on the second insulated wire 34b is removed, and the internal metal wire is connected to the conductive layer 125.

The first insulated wire 34a and the second insulated wire 34b are extended in the longitudinal direction (Z direction) of the support body with the support body 12 interposed between the first insulated wire 34a and the second insulated wire 34b, and do not hinder light emission from the VCSEL. In the arrangement configuration of FIG. 8, the second insulated wire 34b connected to the back surface 122 of the support body 12 may be extended to the vicinity of the tip end of the support body 12 on the + Z side. The first insulated wire 34a and the second insulated wire 34b protruding from the insulating layer 36 in the -Z direction are cooled by coming into contact with the coolant. The support body 12 may also protrude from the insulating layer 36 at at least one end in the longitudinal direction, and may be directly cooled by the coolant. The conductive layers 124 and 125 formed on the surface of the support body 12 also function as a heat sink.

The length of one side and the height of the VCSEL chip used as the light-emitting element 31 are 200 µm or less, and the VCSEL chip is mounted on the support body 12 and sealed with the insulating layer 36. The overall thickness and width of the light irradiation device 30 are about 0.5 mm, and an ultra-small light irradiation device 30 that can be incorporated into the catheter 50 can be realized. In the example of FIG. 8, the thickness of the insulating layer 36 is smaller than the thickness of the light-emitting element 31.

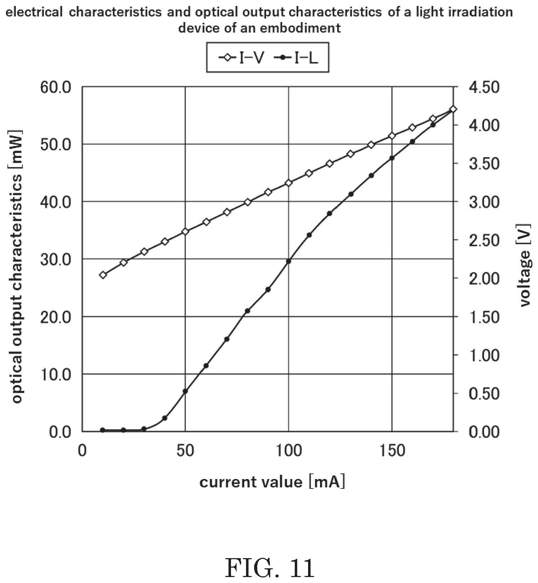

FIG. 11 is a diagram illustrating electrical characteristics and optical output characteristics of a fabricated light irradiation device.

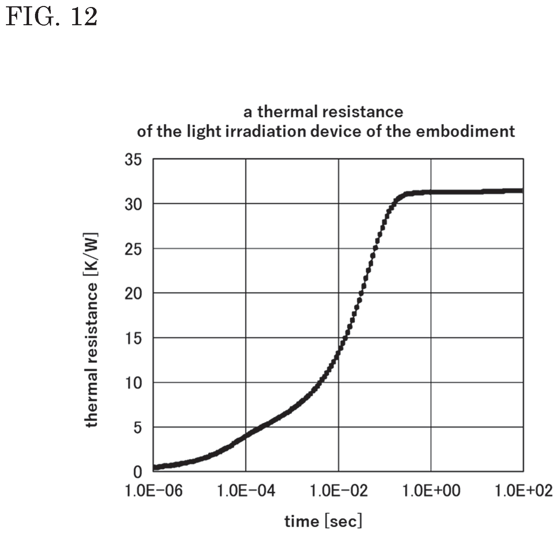

FIG. 12 is a diagram illustrating a thermal resistance. The characteristics of the light irradiation device were measured by fabricating a sample having the configuration of the first embodiment. The light-emitting element 11 used has a resonator length (length in the Z direction) of 1.5 mm, a width (length in the X direction) of 0.2 mm, and an oscillation wavelength of 640 nm. The support body 12 is an AlN substrate having a thickness (length in the Y direction) of 0.1 mm. The mirror is a silver mirror, and the size of the bottom surface (length × width) is 0.3 mm × 0.2 mm, and the height is 0.2 mm. The first insulated wire 14a and the second insulated wire 14b are polyurethane copper wires, each of which has a diameter of 0.1 mm and a length of 1.5 m. The insulating layer 16 is a polysilazane coat.

In FIG. 11, a horizontal axis represents a current value [mA] applied via the insulated wire 14, a vertical axis on the left side represents an optical output [mW], and a vertical axis on the right side represents a voltage [V]. In the drawing, black marks indicate current-to-optical output characteristics, and white marks indicate current-to-voltage characteristics.
The optical output increases with an increase in current. Optical power of 30 mW is obtained by the injected current of 100 mA.

In FIG. 12, a horizontal axis represents time [sec], and a vertical axis represents thermal resistance [K/W]. The thermal resistance is saturated in about 0.1 seconds after the operation of the light irradiation device is turned on. By cooling the light irradiation device with the coolant 51 during operation, heat is efficiently released from the support body 12, the insulated wire 14, and the insulating layer 16, so that the operation of the light irradiation device can be stabilized.

The present disclosure has been described based on the specific configuration examples, but is not limited to the configuration examples described above. For example, in the first embodiment, the second insulated wire 14b that does not hinder light emission may be extended outward from the tip end of the support body 12 in the + Z direction and may function as a heat dissipation member. In the configuration of the first embodiment or the third embodiment, a photodetector such as a photodiode may be disposed together with the light-emitting element on the light-emitting element mounting surface 121 of the support body 12, and may be used as a biological sensor. In the configuration of the third embodiment, instead of mounting the two light-emitting elements 31 on the support body 12, one light-emitting element 31 and one light receiving element may be mounted. When two-core enameled wires are used as the insulated wires 14 and 34, one metal wire may be used for supplying an electrical signal to the light-emitting element 11 or 31, and the other metal wire may be used as a wire for reading a signal output from the photodetector.

The light irradiation devices 10 (including 10A and 10B), 20, and 30 of the first to third embodiments may be used in combination with an endoscope. The light-emitting elements 11 and 31 can be used not only as a laser light source for treatment, but also as a sensing light source or an illumination light source. In any case, the light irradiation device has a heat dissipation property that allows heat from the light-emitting element to be dissipated to the outside, and an electrical insulation property sufficient to secure an insulation property in a coolant, and exhibits an effect as a light irradiation device that can be incorporated into a catheter. Unlike an optical fiber, an insulated wire having an insulating coating is highly flexible. In addition, the configuration in which the light-emitting element itself is mounted on the support body 12 offers high integration, and has a wide range of applications for sensors.

### Industrial Applicability

The present invention can be used not only for a light irradiation device inserted into a medical catheter, but also for other medical applications. The present invention is also applicable to a sensor or the like that requires local light irradiation in combination with a coolant.

Embodiments of the present invention may include configurations described below.
[Aspect 1] A light irradiation device comprising:
   a light-emitting element configured to emit light having a predetermined wavelength;
   a support body on which the light-emitting element is mounted;
   insulated wires electrically connected to the light-emitting element; and
   an insulating layer integrally covering an outer surface of a structure comprising the light-emitting element, the support body, and the insulated wires.
[Aspect 2] The light irradiation device according to aspect 1, further comprising
   an optical component on which the light emitted from the light-emitting element is incident,
   wherein the insulating layer integrally covers an outer surface of a structure comprising the light-emitting element, the support body, the insulated wire, and the optical component.
[Aspect 3] The light irradiation device according to aspect 1 or 2, wherein a thickness of the insulating layer is smaller than a thickness of the light-emitting element.
[Aspect 4] The light irradiation device according to aspect 2, wherein
   the light-emitting element and the optical component are mounted on a first surface of the support body, and
   a light incident surface of the optical component is inclined with respect to the first surface.
[Aspect 5] The light irradiation device according to aspect 2 or 4, wherein the optical component is a reflective member configured to guide an optical axis of the light emitted from the light-emitting element in a direction intersecting a light-emitting element mounting surface of the support body.
[Aspect 6] The light irradiation device according to aspect 2, 4, or 5, wherein a predetermined space is provided between a light-emitting surface of the light-emitting element and the light incident surface of the optical component.
[Aspect 7] The light irradiation device according to any one of aspects 1 to 6, wherein the insulated wires comprise
   a first insulated wire electrically connected to a first electrode of the light-emitting element on a side of the first surface of the support body on which the light-emitting element is mounted, and
   a second insulated wire electrically connected to a second electrode of the light-emitting element on a side of a second surface opposite to the first surface.
[Aspect 8] The light irradiation device according to aspect 7, wherein the support body has a lateral surface connecting the first surface and the second surface, and a conductive layer formed on at least a part of the second surface, and
   the second insulated wire is connected to the second electrode of the light-emitting element via the conductive layer.
[Aspect 9] The light irradiation device according to aspect 2, 4, 5, or 6, wherein the optical component is a mirror.
[Aspect 10] The light irradiation device according to aspect 9, wherein the mirror comprises a reflective layer made of metal and/or a dielectric.
[Aspect 11] The light irradiation device according to any one of aspects 1 to 10, wherein the insulating layer is biocompatible.
[Aspect 12] The light irradiation device according to any one of aspects 1 to 11, wherein the insulating layer comprises a polysilazane coat.
[Aspect 13] An in-vivo light irradiation assembly comprising:
   a catheter; and
   the light irradiation device according to any one of aspects 1 to 12, the light irradiation device being inserted into the catheter,
   wherein an inside of the catheter is filled with a coolant at least during use.

This application claims priority based on Japanese Patent Application No. 2023-211661, filed with the Japan Patent Office on December 15, 2023, the entire contents of which are included herein by reference.

### Reference Signs List

10, 10A, 10B, 20, 30 Light irradiation device
11, 31 Light-emitting element
111 Light-emitting surface
12, 12-1, 12-2 Support body
121 Light-emitting element mounting surface
122 Back surface
124, 125 Conductive layer
14, 24, 34 Insulated wire
14a, 24a, 34a First insulated wire
14b, 24b, 34b Second insulated wire
16, 16A, 16B, 26, 36 Insulating layer
17, 27 Optical component
50 Catheter
51 Coolant
100, 200 In-vivo light irradiation assembly

## Claims

1. A light irradiation device comprising:
a light-emitting element configured to emit light having a predetermined wavelength;
a support body on which the light-emitting element is mounted;
insulated wires electrically connected to the light-emitting element; and
an insulating layer integrally covering an outer surface of a structure comprising the light-emitting element, the support body, and the insulated wires.

2. The light irradiation device according to claim 1, further comprising
an optical component on which the light emitted from the light-emitting element is incident,
wherein the insulating layer integrally covers an outer surface of a structure comprising the light-emitting element, the support body, the insulated wire, and the optical component.

3. The light irradiation device according to claim 1 or 2, wherein a thickness of the insulating layer is smaller than a thickness of the light-emitting element.

4. The light irradiation device according to claim 2, wherein
the light-emitting element and the optical component are mounted on a first surface of the support body, and
a light incident surface of the optical component is inclined with respect to the first surface.

5. The light irradiation device according to claim 2 or 4, wherein the optical component is a reflective member configured to guide an optical axis of the light emitted from the light-emitting element in a direction intersecting a light-emitting element mounting surface of the support body.

6. The light irradiation device according to claim 2, 4, or 5, wherein a predetermined space is provided between a light-emitting surface of the light-emitting element and the light incident surface of the optical component.

7. The light irradiation device according to any one of claims 1 to 6, wherein the insulated wires comprise
a first insulated wire electrically connected to a first electrode of the light-emitting element on a side of the first surface of the support body on which the light-emitting element is mounted, and
a second insulated wire electrically connected to a second electrode of the light-emitting element on a side of a second surface opposite to the first surface.

8. The light irradiation device according to claim 7, wherein the support body has a lateral surface connecting the first surface and the second surface, and a conductive layer formed on at least a part of the second surface, and
the second insulated wire is connected to the second electrode of the light-emitting element via the conductive layer.

9. The light irradiation device according to claim 2, 4, 5, or 6, wherein the optical component is a mirror.

10. The light irradiation device according to claim 9, wherein the mirror comprises a reflective layer made of metal and/or a dielectric.

11. The light irradiation device according to any one of claims 1 to 10, wherein the insulating layer is biocompatible.

12. The light irradiation device according to any one of claims 1 to 11, wherein the insulating layer comprises a polysilazane coat.

13. An in-vivo light irradiation assembly comprising:
a catheter; and
the light irradiation device according to any one of claims 1 to 12, the light irradiation device being inserted into the catheter,
wherein an inside of the catheter is filled with a coolant at least during use.
